Europäisches Patentamt

European Patent Office

Office européen des brevets

(19)

(11) Veröffentlichungsnummer: **0 214 101**
**A2**

(12) **EUROPÄISCHE PATENTANMELDUNG**

(21) Anmeldenummer: **86810386.2**

(22) Anmeldetag: **28.08.86**

(51) Int. Cl.⁴: **A 61 K 31/16**
**A 61 K 31/44**

(30) Priorität: **03.09.85 CH 3783/85**

(43) Veröffentlichungstag der Anmeldung:
**11.03.87 Patentblatt 87/11**

(84) Benannte Vertragsstaaten:
**BE CH DE FR GB IT LI LU NL SE**

(71) Anmelder: **CIBA-GEIGY AG**
**Klybeckstrasse 141**
**CH-4002 Basel(CH)**

(72) Erfinder: **Jung, Albrecht, Dr.**
**Hausserstrasse 142**
**D-7400 Tübingen(DE)**

(72) Erfinder: **Vosbeck, Klaus Dieter, Dr.**
**13049 Caminito Mar Villa**
**Del Mar California 92014(US)**

(54) **Verwendung von Eisen(III)-Chelatoren vom Typ Desferrioxamin B und Desferriferrithiocin zur Behandlung von Malaria.**

(57) Humane Malaria, insbesondere Malaria tropica mit Plasmodium falciparum als Erreger, kann durch Verabreichung von Eisen(III)-Chelatoren der Klasse Desferrioxamin B und Desferriferrithiocin erfolgreich behandelt werden, wobei Chloroquin-resistente Plasmodium-Stämme gegen die Chelatoren besonders empfindlich sind.

EP 0 214 101 A2

0214101

CIBA-GEIGY AG

4-15487/=

Basel (Schweiz)

BEZEICHNUNG GEÄNDERT
siehe Titelseite

Behandlung von Malaria

Malaria ist allgemein bekannt als eine gefährliche Infektionskrankheit, welche durch gewisse Stechmückenarten (Moskitos) der
Gattung Anopheles übertragen wird und sich durch periodisch wiederholte, mit Schüttelfrost verbundene Hochfieberanfälle von 40-41°C
manifestiert. Malaria ist in verschiedenen Formen weltweit verbreitet in feuchtwarmen Gebieten, insbesondere jenen der subtropischen und tropischen Zonen, und ihre derzeitige Ausbreitung
wird auf etwa 200-400 Millionen Fälle geschätzt. Allein in Afrika
rechnet man bei Malaria-Erkrankungen in etwa 10 % der Fälle mit
direktem tödlichem Ausgang; in den übrigen Fällen hat sie eine
beträchtliche langdauernde Schwächung zur Folge. Bei Kleinkindern
wird sie als Ursache in etwa 50 % aller Todesfälle verantwortlich
gehalten. Jahrzehnte, sogar Jahrhunderte trotzt die Seuche allen
Versuchen um ihre Bannung und stellt nach wie vor eines der grossen
Probleme des Weltgesundheitswesens dar. Abgesehen von grossangelegten umfassenden Bemühungen um gesamthafte oekologische Lösung
(wie Bekämpfen der Stechmücken durch Insekticide oder, noch tiefgreifenden, durch Entwässern und Austrocknen der Sümpfe), stehen im
Vordergrund des Interesses die Möglichkeiten der medizinischen
Behandlung des Menschen als Träger, darunter auch insbesondere die
Suche nach therapeutischen Mitteln zur unmittelbaren Bekämpfung der
Krankheitserreger.

In der Tat ist Malaria eine Gesamtbezeichnung für mehrere Krankheiten, die mit ähnlichem Krankheitsbild verlaufen, aber deren Erreger - parasitäre Protozoen aus der Klasse Sporentierchen - verschiedene Arten von Plasmodium sind. Diese Erkrankungen treten nicht nur beim Menschen, sondern auch bei Säugetieren auf, wobei sie Art-spezifisch sind, d.h. von einer Tierart auf eine andere oder auf Menschen nicht übertragen werden können. Beim Menschen unterscheidet man dem Krankheitsbild nach 3 Formen: Malaria tertiana (Erreger Plamodium vivax und P. ovale), M. quartana (P. malariae) und die besonders schwere und bösartige M. tropica (P. immdaculatum oder falciparum). Die medizinische Behandlung und Medikamente sind allerdings in allen 3 Fällen dieselben.

Charakteristisch für alle Plasmodien-Arten ist ihr komplizierter Entwicklungszyklus, dessen geschlechtliche Vermehrungsstadien im Darmtrakt weiblicher Stechmücken (als Ueberträger) sich abspielen und in ihrer Speicheldrüse zur Uebertragung bereitgehalten werden, wogegen die enorme ungeschlechtliche Vermehrung in der Leber und den roten Blutkörperchen des Menschen als Träger stattfindet. Auf den Menschen wird die Infektion beim Mückenstich übertragen, wobei die winzigen einkernigen Sichelkeime (Sporozoiten) des Parasiten mit dem Speichel des Ueberträgers eingeimpft werden und auf dem Blutwege in die Leber des Trägermenschen gelangen. Nach Eindringen in die Leberzelle wächst ungeschlechtlich der Sporozoit zu einem vielkernigen Gebilde (Schizonten) heran, durch dessen Zerfall etwa 5000-10 000 einkernige Sprosszellen (Merozoiten) freigesetzt werden. Diese können zur weiteren Fortpflanzung wiederum die Leberzellen befallen, oder die Erythrozyten (rote Blutkörperchen) angreifen. Jeder Merozoit dringt in ein Blutkörperchen ein, baut dessen Zellmasse weitgehend ab und verwendet die gewonnenen Bausteine - vorwiegend Aminosäuren - für eigene Stoffwechselprozesse und Energiegewinnung. Dadurch bildet er in einem extram raschen ungeschlechtlichen Vermehrungsvorgang (Schizogonie) innerhalb von 48 (bzw. 72) Stunden 10-20 neue Merozoiten und zerstört dabei völlig die Wirtszelle. (Mit derartigen Wachstumsraten übertreffen Plasmodien selbst schnellwachsende Tumoren.) Die entstandenen

Merozoiten befallen weitere Erythrozyten zu einem neuen Vermehrungsstadium. Dieser zyklische Verlauf ist für die wohlbekannten Malaria-
Symptome verantwortlich, da insbesondere die Blutzerfallsprodukte
den hohen Fieber und Schüttelfrost verursachen und die typischen
(fast) fieberfreien Perioden von 48 (bei M. tertiana und M. tropica)
bzw. 72 (bei M. quartana) dem Vermehrungsvorgang des Parasiten im
Blut zeitlich entsprechen. Nach mehrmaliger Wiederholung lässt die
Intensität (nicht aber der Zeitabstand!) der Anfälle allmählich bis
zum völligen Abklingen nach. Wahrscheinlich spielt dabei einerseits
die natürliche Erschöpfung und Abnahme der Vitalität der Erregerzellen, andererseits die Entwicklung einer Art Immunität des Trägers
eine Rolle.

Wie andere intrazelluläre Parasiten, stellt auch Plasmodium für die
Therapie ein besonderes Problem dar: abgesehen von den verschiedenen
Entwicklungsformen ist der Mikroorganismus fast nie einem direktem
Angriff eines exogenen oder endogenen (d.h. durch den Träger
produzierten) Inhibitors zugänglich, da er meistenzeits in Wirtszellen Unterschlupf findet und somit durch 2 funktionell sehr
verschiedene Zellmembranen zweier physiologisch weit auseinanderliegenden Zellarten (der eigenen und derjenigen der Wirtszelle)
geschützt wird. Die funktionellen Eigenschaften der Erythrozytenmembran sperren den Zugang zum Parasiten für eine Reihe von
Inhibitoren (wie körpereigenen Immunfaktoren), welche, wenn er
extrazellulär leben würde, sicherlich gut wirksam wären. Wahrscheinlich aus diesem Grunde haben bisher auch alle Versuche gescheitert,
die Krankheit durch immunologische Behandlungsmethoden einschliesslich Schutzimpfung unter Kontrolle zu bringen. Somit bleiben
Chemotherapeutika unter den Antimalariamitteln immer noch am
erfolgreichsten, wie z.B. das klassische Chinin, welches für
Jahrhunderte konkurrenzlos das einzige wirksame Heilmittel war und
trotz gewisser Nachteile weiterhin eine bedeutende Stelle in der
Malaria-Therapie einnimmt. Im Laufe der letzten 50 Jahre wurden
zahlreiche Chemotherapeutika verschiedenster Strukturklassen als
Schizontozide entwickelt, d.h. Wirkstoffe, die in den Entwicklungszyklus der Plasmodien in einem ihrer ungeschlechtlichen Vermehrungsstadien eingreifen und die Teilung entweder im Lebergewebe oder in
Erythrocyten unterbinden, oder sogar die Merozoiten töten. Präparate
der ersten Gruppe (Gewebe-Schizontozide) sind beispielsweise durch
Primaquin [6-Methoxy-8-(4-amino-1-methylbutylamino)chinolin]
repräsentiert; stellvertretend für die Gruppe der Blut-Schizontozide
ist neben Chinin auch das bisher erfolgreichste Antimalarikum
Chloroquin [7-Chlor-4-(4-diethylamino-1-methylbutylamino)chinolin]
hervorzuheben. (Eine Zusammenstellung wichtigerer bekannter Chemotherapeutika ist in der Fachliteratur zu finden, vgl. Advances in
Malaria Chemotherapy, WHO Technical Report Series 711; World Health
Organisation, Geneva, 1984; Seiten 12, 13 und 163-175.

Die allgemeine Anwendbarkeit dieser Heilmittel vermindert sich
jedoch im Laufe der Zeit infolge der ausgeprägten Fähigkeit von
Plasmodien, Resistenz gegen Chemotherapeutika zu entwickeln. Solche
Resistenz wurde bereits Anfangs des 20. Jahrhunderts beim Chinin
beobachtet. Zurzeit betrifft sie in immer steigendem Masse
Chloroquin und intensiviert somit die Suche nach neuen Lösungen.

Die heutige tiefere Einsicht in die metabolischen Vorgänge im
Entwicklungszyklus von Plasmodien lenkte Aufmerksamkeit darauf, dass
die enorm schnelle Vermehrung der Zellpopulation, sowie deren
Ueberleben, unmittelbar von einer ausreichenden Versorgung mit Eisen
abhängig sein muss. Dies gilt insbesondere für die entsprechend
hohe DNA-Synthese, in welcher das eisenhaltige Enzym Ribonucleotidreductase eine Schlüsselrolle spielt. Zur Sicherung der Vermehrung
von Plasmodien muss Eisen in die Parasitenzelle über ein geeignetes Transportsystem gelangen. Dazu muss es unter Umständen
nicht nur durch die Zellmembran des Parasiten, sondern auch, falls
dieses es dem Erythrozyten als Wirtszelle nicht entnehmen kann,
durch die phylogenetisch verschiedene Zellmembran des Wirtes. Wenn
es nun gelingt, die Verfügbarkeit des Eisens für das Enzym zu
unterbinden, z.B. durch eine feste Bindung an einen Komplexbildner,
so könnte man den Vermehrungsmechanismus zum Stillstand bringen oder
sogar die Parasitenzelle töten.

Einem derartigen Mechanismus ist offensichtlich die antibakterielle Wirkung von gewissen aus natürlichen Quellen, insbesondere aus Mikroorganismen, gewonnenen Eisen(III)-Chelatoren zuzuschreiben. Eine gewisse allgemeine antibakterielle Wirksamkeit in vitro wurde z.B. beim Desferri-ferrithiocin [2-(3-Hydroxy-2-pyridyl)-4-methyl-2-thiazolin-4-carbonsäure] und seinen halbsynthetischen Derivaten der Europäischen Patentschrift 0045281, sowie beim Desferrioxamin B [6,17,28-Trihydroxy-7,10,18,21,29-pentaoxo-6,11,17,22,28-pentaaza-triacontanylamin), vgl. H. Bickel, H. Keberle und E. Vischer: Helv. Chim. Acta 46, 1385-9 [1963]. Bei Bakterien allerdings handelte es sich (abgesehen davon, dass lediglich die in vitro--Wirksamkeit nachgewiesen wurde) um den einfachen Fall, in welchem das Eisen(III)-komplexierende Mittel lediglich eine einzige, nämlich die bakterielle Membran durchdringen musste.

Im vorliegenden Fall der Parasitenzellen eines Plasmodiums hängt aber der Erfolg davon ab, ob der Komplexbildner nicht nur durch die Zellmembran des Parasiten, sondern auch durch die phylogenetisch ganz verschiedene Zellmembran des Erythrozyten als Wirtszelle transportierbar ist. Glücklicherweise ist diese Bedingung bei den oben erwähnten Eisen(III)-Chelatoren natürlichen Ursprungs und ihren halbsynthetischen Analogen erfüllt, und es konnte experimentell nachgewiesen werden, dass verschiedene Plasmodium-Arten in vitro, darunger auch Plasmodium falciparum in Kultur an menschlichen Erythrozyten, durch Desferrioxamin B nicht nur in ihrem Wachstum gehemmt, sondern auch getötet werden. Auch in Versuchen in vivo mit einer für Nagetiere spezifischen Plasmodium-Art (P. vinckei) wurde nachgewiesen, dass Desferrioxamin B bei 0,2 g/kg das Wachstum des Parasiten deutlich hemmt, und bei 1,0 g/kg ihn vollständig tötet, vgl. A. Jung et al.; Abstracts of Iron Club Meeting, Rennes (France), July 10-13, 1984. Da die Wirkung von Desferrioxamin B im Tierversuch und in der humanen Applikation bereits in einem anderen Zusammenhang korreliert wurden, und da auch ähnliche Korrelation zwischen Plasmodium vinckei in der Maus und P. falciparum im Mensch bei klassischen Antimalarika bekannt sind, sind somit auch theoretische Grundlagen gegeben, die Möglichkeit und Bedingungen für eine therapeutische Anwendung am Menschen auszuwerten. Wahrscheinlich ist man durch eine solche Analyse zum Schluss gekommen, dass die notwendige Dosierung des Heilmittels (obwohl es auch in sehr hohen Dosen toxikologisch einwandfrei ist) unpraktisch und für breitere Anwendung uninteressant wäre - jedenfalls steht fest, dass gegen therapeutische Anwendung von Desferrioxamin B oder analogen Eisenchelatoren bei humaner Malaria offensichtlich ein Vorurteil herrschte und diese nie ausprobiert worden ist.

Die ungünstige Situation hat sich durch die vorliegende Erfindung schlagartig geändert, indem sich gezeigt hat, dass die oben erwähnten Eisenchelatoren überraschenderweise gegen Chloroquinresistente Stämme von Plasmodium falciparum bei wesentlich niedrigerer Dosierung als bei normalen Stämmen wirksam sind, also gerade gegen die Parasiten, welche durch ihre Resistenz gegenüber konventionellen Chemotherapeutika als eines der schwierigsten Probleme im Kampfe gegen Malaria angesehen werden.

Durch einen experimentellen Vergleich der Wirksamkeit von Desferrioxamin B gegen P. falciparum in Kultur an menschlichen Erythrozyten wurde gezeigt, dass das Chemotherapeutikum um fast eine Grössenordnung wirksamer das Wachstum eines Chloroquin-resistenten Stammes als eines unter gleichen Bedingungen gezüchteten, gegen Chloroquin normal empfindlichen Stammes hemmt.

Gegenstand der vorliegenden Erfindung ist eine pharmazeutische Zusammensetzung zur Behandlung der humanen Malaria, gekennzeichnet durch den Gehalt einer gegen die Krankheitserreger wirksamen Dosis eines Eisen(III)-Chelators der Klasse Desferrioxamin B, Desferri-ferrithiocin und deren pharmakologisch anwendbaren Derivate, gegebenenfalls im Gemisch mit konventionellen pharmazeutischen Träger- und Hilfsstoffen. Gegenstand der Erfindung ist auch die Verwendung eines Eisen(III)-Chelators der Klasse Desferrioxamin B, Desferriferrithiocin und deren pharmakologisch anwendbaren Derivate zur Herstellung einer solchen oben definierten pharmazeutischen Zusammensetzung sowie das Verfahren zur Herstellung derselben.

Gegenstand der Erfindung ist auch die Verwendung eines Eisen(III)-Chelators der Klasse Desferrioxamin B, Desferriferrithiocin und deren pharmakologisch anwendbaren Derivate zur Behandlung der humanen Malaria sowie die entsprechende therapeutische Methode, gekennzeichnet durch die Verabreichung des obengenannten Wirkstoffes in einer gegen die Krankheitserreger wirksamen Dosis allein oder in Form einer geeigneten pharmazeutischen Zusammensetzung, wie insbesondere der oben definierten.

Unter den zu behandelnden Malaria-Erkrankungen sind Infektionen durch Plasmodien-Arten zu verstehen, z.B. durch Stämme von P. vivax oder P. ovale (M. tertiana), P. malariae (M. quartana) und insbesondere P. falciparum oder P. immaculatum (M. tropica), sowie gemischte Infektionen mit 2 oder mehreren Stämmen derselben oder einer anderen Plasmodium-Art oder auch Reinfektionen mit demselben Stamm, jedoch in einer zeitlich verschobenen Phase. Insbesondere betreffen alle oben angegebenen Aspekte der Erfindung die Behandlung von Malaria tropica mit Plasmodium falciparum als Erreger. Die oben angegebenen Aspekte betreffen auch insbesondere die Behandlung von Malaria-Erkrankungen durch Plasmodium-Stämme, welche gegen die konventionellen Chemotherapeutika, wie insbesondere Chinin und vor allem Chloroquin, sowie andere wirkungsanaloge Heilmittel, resistent sind, und darunter ganz besonders die Behandlung der Krankheit in ihrer akuten Phase, nämlich während der Vermehrung von Plasmodien in Erythrozyten des Trägers.

Ein erfindungsgemäss anzuwendender Eisen(III)-Chelator ist in erster Linie das oben erwähnte Desferrioxamin B als freie Base oder auch als eines seiner bekannten pharmazeutisch brauchbaren Säureadditionssalze, wie Hydrochlorid und insbesondere Methansulfonat (Mesylat), vgl. The Merck Index, Item 2839, Seite 412; 10. Edition, Merck Co., Inc., Rahway, N.J., U.S.A.; 1983. Als Arzneimittel zur erfindungsgemässen Behandlung von Malaria wird es insbesondere in zur parenteralen Verabreichung geeigneter Form, wie z.B. als wässrige Lösung, zur Verfügung gestellt. Vorzugsweise wird das Arzneimittel, insbesondere ein geeignetes pharmazeutisch brauchbares Säureadditionssalz von Desferroxamin B, z.B. das Mesylat, in einer zur parenteralen Verabreichung von etwa 0,2 bis 5 g täglich, vor allem von etwa 0,2 bis etwa 1,5 g und in erster Linie etwa 0,5 bis etwa 1,0 g täglich, zur Verfügung gestellt. Die Dosierung richtet sich nach dem Gewicht und individuellem Zustand des Patienten und vor allem der Empfindlichkeit des Krankheitserregers; besonders zweckmässig ist die Verabreichung von zweimal täglich 0,5 g, welche bis zum Verschwinden aller Symptome währen 1-2 Wochen fortgesetzt wird. In Anfangsstadien, und besonders bei Anfällen kann diese Tagesdosis bis auf das Vierfache erhöht werden.

Geeignete Formulierungen zur Behandlung von Malaria sind insbesondere 2,5 bis etwa 20%ige, vorzugsweise etwa 5 bis etwa 10%ige, wässrige Lösungen von pharmazeutisch brauchbaren Säureadditionssalzen von Desferrioxamin B, wobei diese Lösungen unmittelbar oder kurz vor der Verabreichung aus entsprechenden Trockenampullen, oder aus anderen stabilen und steril gelagerten Formulierungen des Salzes und aus destilliertem oder entmineralisiertem Wasser hergestellt werden. Beispielsweise können 10%ige oder selbst geringer konzentrierte Lösungen von Desferrioxaminmesylat hergestellt werden unter Verwendung handelsüblicher 500 mg Trockenampullen von DESFERAL® (Warenzeichen der Ciba-Geigy AG) und zum Beispiel destilliertem Wasser oder physiologischer Natriumchloridlösungen. Derartige Lösungen können parenteral verabreicht werden, z.B. durch intramuskuläre Injektion, z.B. in den Glutaeus maximus, oder in die vorderen Oberschenkelmuskel oder den Deltoideusmuskel, sowie auch durch intravenöse oder subkutane Injektion oder durch Infusion. Geeignet insbesondere zur Selbstverabreichung oder zur Verabreichung durch nicht geschulte Personen, z.B. durch Gesundheitshelfer in Entwicklungsländern, ist eine langsame subkutane Injektion, z.B. in die abdominale Haut, unter Verwendung einer tragbaren Infusionspumpe und einer flexibel befestigten Injektionsspritze, über den Verlauf mehrerer Stunden einmal oder zweimal täglich oder kontinuierlich während des Tages oder der Nacht, derart, wie sie zur bekannten Behandlung von Krankheiten wie Thalassämie, ausgeübt wird.

Die Herstellung von Desferrioxamin B und von pharmazeutisch brauchbaren Salzen, insbesondere dem Hydrochlorid, wird in der DE-PS 1 186 076 beschrieben. Darüber hinaus werden Salze, wie das bereits genannte Mesylat (Methansulfonat), Sulfat und Tartrat, in der FR-PS 1 898 M beschrieben. In dieser Veröffentlichung wird auch die Herstellung pharmazeutischer Formulierungen, z.B. von Trockenampullen, Suppositorien und Kapseln, die solche Salze enthalten, beschrieben. Weitere geeignete Salze sind solche, wie sie in der DE-PS 1 186 076 beschrieben werden, einschliesslich beispielsweise solcher mit Phosphorsäuren, Essigsäure, Glykolsäure, Milchsäure, Bernsteinsäure, Maleinsäure, Fumarsäure, Apfelsäure, Weinsäure, Zitronensäure, Ethansulfonsäure und Hydroxyethansulfonsäure.

Ein erfindungsgemäss anzuwendender Eisen(III)-Chelator ist ebenfalls das oben erwähnte Desferri-ferrithiocin und dessen Derivate, wie insbesondere pharmazeutisch brauchbare Salze, vor allem Alkalimetall- und Erdalkalimetallsalze, und pharmakologisch wirksame Analoga, wie insbesondere Niederalkanester, welche in der EP-PS 0045281 beschrieben sind.

Geeignete Formulierungen von Wirkstoffen dieses Typs sind solche zur enteralen, wie oralen, sowie parenteralen, wie subkutanen, Verabreichung, wobei sie den Wirkstoff allein oder im Gemisch mit einem pharmazeutisch anwendbaren Trägermaterial enthalten. Die Dosierung ist etwa dieselbe wie die oben angegebene, auch sie hängt vom Alter, Gewicht und individuellem Zustand des Kranken, von der Empfindlichkeit des Erregers, sowie von der Applikationsweise ab. - Die pharmazeutischen Präparate enthalten von etwa 10 % bis etwa 95 %, vorzugsweise von etwa 20 % bis etwa 90 % (Gewicht/Gewicht) des Wirkstoffes bei festen Formen, von etwa 5 % bis etwa 20 %, vorzugsweise etwa 10 % bei flüssigen Applikationsformen, und können in Dosiseinheitsform, wie Dragées, Tabletten, Kapseln, Suppositorien oder auch Ampullen und Trockensubstanz-Ampullen, vorliegen.

**0214101**

Die pharmazeutischen Präparate der vorliegenden Erfindung werden in an sich bekannter Weise, z.B. mittels konventioneller Misch-, Granulier-, Mahl-, Lösungs- oder Lyophilisierungsverfahren, hergestellt. Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Die folgenden Beispiele dienen zur Illustration der Erfindung.

Beispiel 1: Trockenampullen mit einem Gehalt von 250 mg aktivem Bestandteil zum Zubereiten von 10%igen oder 5%igen (G/V) wässrigen Injektions-Lösungen durch Zusatz der entsprechenden Menge an sterilisiertem Wasser werden hergestellt, indem man 2,5 ml einer 10%igen (G/V) Lösung von Desferrioxamin-B-mesylat in Ampullen für 2,5 bzw. 5 ml gebrauchsfertige Lösung abfüllt und in üblicher Weise lyophilisiert.

In analoger Weise werden Trockenampullen, die 500 mg Desferrioxamin-B-mesylat enthalten, zur Bereitung von 5,0 ml einer 10%igen (G/V) wässrigen Lösung oder 2,5 ml einer 20%igen (G/V) wässrigen Lösung des aktiven Bestandteils in Ampullen für 5 ml abfüllt und lyophilisiert.

Die für das Lyophilisieren verwendeten Lösungen können zusätzlich 8 % (G/V) Mannit, entsprechend 200 mg bzw. 400 mg pro Ampulle, enthalten.

Beispiel 2: Herstellung von 1000 Kapseln mit 260 mg des Wirkstoffes pro Kapsel

Zusammensetzung

| | |
|---|---|
| Desferriferrithiocin | 260 g |
| Talk | 36 g |
| Weizenstärke | 24 g |
| Magnesiumstearat | 16 g |
| Laktose | 4 g |
| | 340 g |

Zubereitung

Die pulverförmigen Substanzen werden durch ein Sieb mit einer
Maschenweite von 0,6 mm getrieben und gründlich gemischt. Mit je
340 mg dieser Mischung werden mit einer Kapselfüllmaschine Gelatine-
Kapseln bereitet.

Beispiel 3: Herstellung von 1000 Kapseln enthaltend 105 mg des
Wirkstoffes pro Kapsel

Zusammensetzung

| | |
|---|---:|
| Desferriferrithiocin | 105 g |
| Ethylcellulose | 3 g |
| Stearinsäure | 3 g |
| | 111 g |

Zubereitung

Die Ethylcellulose und die Stearinsäure werden in 120 ml Methylenchlorid gelöst, mit dem Wirkstoff versetzt, und die Masse wird durch
ein Sieb von 0,6 mm Maschenweite bei einer Temperatur von ca. 40°
geschlagen, wobei das Methylenchlorid verdampft. 111 mg des erhaltenen Granulates werden in Gelatine-Kapseln zu 0,5 ml mit Hilfe
einer Kapsel-Abfüllmaschine abgefüllt.

Versuchsbericht

Testmikroorganismen

a) Durch konventionelles Züchten des Stammes Plasmodium
falciparum T96 in einer 2 % (V/V) Suspension von humanen
Erythrozyten wird der Grundorganismus-Zellinie A fortgepflanzt,
welche eine normale Empfindlichkeit gegenüber Chloroquin aufweist.

b) Zellinie A des obigen Stammes wird unter analogen Standardbedingungen wie unter a) gezüchtet, jedoch mit Zusatz von immer
ansteigenden Konzentrationen von Chloroquin, bis eine Chloroquin-

resistente <u>Zellinie B</u> resultiert, welche weiterhin in Kulturmedien mit einer 500 nM-Konzentration von Chloroquin andauernd normales Wachstum (dieselbe Wachstumsraten wie Zellinie A) aufweist.

Versuchsanordnung:

I. Die Züchtung von Testorganismen erfolgt unter den für Züchtung von Plasmodien üblichen Bedingungen auf einer 2 % Suspension von humanen Erythrozyten, supplementiert mit 50 mg/l Hypoxanthin und gegebenenfalls unter Zugabe der getesteten Hemmsubstanz in gewünschter Konzentration. Die initiale Parasitämie (= der prozentuelle Anteil der zu Beginn von Plasmodien infizierten Erythrozyten) beträgt 1-2 %. Das Resultat (Hemmwirkung) wird als $IC_{50}$ ausgewertet, d.h. jene Konzentration des Wirkstoffes, die eine Senkung der finalen Parasitämie um 50 %, d.h. eine 50%ige Hemmung des Wachstums, verursacht.

II. In einer alternativen Versuchsanordnung wird beim Einhalten aller übrigen Bedingungen <u>ohne</u> Zugabe von Hypoxanthin und mit einer initialen Parasitämie von 0,5 % gearbeitet.

<u>Vorversuche</u> (Empfindlichkeit gegenüber Chloroquin)

Zellinie A: Unter Versuchsanordnung I ohne Chloroquin vermehren sich die Plasmodien innerhalb von 48 Stunden um einen Faktor von 7-9; $IC_{50}$ = ~50 nM Chloroquin über 2 Zyklen, was einer normalen Chloroquin-Empfindlichkeit anderer Stämme entspricht.
Unter Versuchsanordnung II beträgt $IC_{50}$ etwa 10-15 nM Chloroquin über 2 Zyklen.

Zellinie B: Unter Versuchsanordnung I mit 500 nM-Konzentration von Chloroquin beträgt der Vermehrungsfaktor wiederum 7-9 (d.h. ist demjenigen von Zellinie A <u>ohne</u> Chloroquin gleich!); $IC_{50}$ = ~800 nM Chloroquin über 2 Zyklen.

<u>Hauptversuch</u> (Empfindlichkeit gegenüber Desferrioxamin B)

Zellinie A: Versuchsanordnung I: $IC_{50}$ = 17 ± 3 µM Desferrioxamin B über 2 Zyklen.

Versuchsanordnung II: $IC_{50}$ = 5 ± 2 µM Desferrioxamin B über 2 Zyklen.


Zellinie B: a) ohne Chloroquin-Zusatz im Testmedium

Versuchsanordnung I: $IC_{50}$ = 6 ± 1,5 µM Desferrioxamin B über 2 Zyklen.

Versuchsanordnung II: $IC_{50}$ = 2 ± 1 µM Desferrioxamin B über 2 Zyklen.


b) mit Zugabe von Chloroquin in einer 400 nM-Konzentration (d.h. unter der Resistenzschwelle)

Versuchsanordnung I: $IC_{50}$ = 3 ± 1 µM Desferrioxamin B über 2 Zyklen.

Versuchsanordnung II: $IC_{50}$ = 0,6-0,8 ± 0,1 µM Desferrioxamin B über 2 Zyklen.

Patentansprüche

1. Verwendung eines Eisen(III)-Chelators der Klasse Desferrioxamin B, Desferriferrithiocin und deren pharmakologisch wirksamen Derivate zur Behandlung der humanen Malaria, gekennzeichnet durch die Verabreichung einer gegen Malaria-Erreger wirksamen Dosis des obgenannten Wirkstoffes allein oder in Form einer pharmazeutischen Zusammensetzung.

2. Verwendung gemäss Anspruch 1, dadurch gekennzeichnet, dass der Eisen(III)-Chelator Desferrioxamin B in Form freier Base oder eines pharmazeutisch verwendbaren Säureadditionssalzes ist.

3. Verwendung gemäss Anspruch 1, dadurch gekennzeichnet, dass der Eisen(III)-Chelator Desferriferrithiocin in Form freier Säure, eines pharmazeutisch verwendbaren Alkalimetall- oder Erdalkalimetallsalzes oder eines Niederalkylesters ist.

4. Verwendung gemäss einem der Ansprüche 1-3, dadurch gekennzeichnet, dass man den Wirkstoff in einer täglichen Dosis von etwa 0,2 bis etwa 10 g, bezogen auf einen Patienten von 70 kg Gewicht, verabreicht.

5. Verwendung gemäss Anspruch 4, dadurch gekennzeichhnet, dass man den Wirkstoff in akuten Phasen der Krankheit in einer täglichen Dosis von etwa 1 bis etwa 8 g, in Ruhephasen von etwa 0,5 bis 2 g, bezogen auf einen Patienten von 70 kg Gewicht, verabreicht.

6. Verwendung gemäss einem der Ansprüche 1-5, dadurch gekennzeichnet, dass man die Malaria tropica mit Plasmodium falciparum als Erreger behandelt.

7. Verwendung gemäss Anspruch 6, dadurch gekennzeichnet, dass die Malaria tropica durch einen gegen Antimalarika resistenten Stamm von Plasmodium falciparum verursacht wird.

8. Verwendung gemäss Anspruch 7, dadurch gekennzeichnet, dass die Malaria tropica durch einen gegen Chloroquin resistenten Stamm von Plasmodium falciparum verursacht wird.

9. Pharmazeutische Zusammensetzung zur Verwendung gemäss einem der Ansprüche 1-8, gekennzeichnet durch den Gehalt des dort definierten Wirkstoffes zusammen mit mindestens einem pharmazeutischen Hilfsmaterial.

10. Verwendung eines Eisen(III)-Chelators der Klasse Desferrioxamin B, Desferriferrithiocin und deren pharmakologisch wirksamen Derivate zur Herstellung einer pharmazeutischen Zusammensetzung zur Anwendung als Antimalarikum.

FO 7.4 SY/eg*